# EUROPÄISCHE PATENTANMELDUNG

(11) **EP 1 609 763 A1**
(43) Veröffentlichungstag der Anmeldung: **28.12.2005**
(21) Anmeldenummer: 05012036.9
(22) Anmeldetag: 03.06.2005
(51) Int. Cl.: C02F 3/28, C02F 11/04

(54) **Bioreaktor**

(30) Priorität: 24.06.2004 DE 102004030559
(71) Anmelder: LINDE-KCA-Dresden GmbH, 01277 Dresden (DE)
(72) Erfinder: Langhans, Gerhard, Dr., 01159 Dresden (DE)

(57) **Zusammenfassung**

Es wird ein Bioreaktor zur Behandlung von organikhaltigem Schlammsuspensionen wie z. B. Bioabfällen oder Güllen beschrieben. Der Bioreaktor besteht aus einem Behälter 1 mit einem Behälterboden 2 und einem im zentralen Bodenbereich angebrachten Feststoffabzug 9. Um ein Zusetzen des Bioreaktors durch sedimentierte partikuläre Feststoffe zu verhindern, wird vorgeschlagen im Bodenbereich einen nicht tragenden, konischen Einsatz 6 vorzusehen, welcher eine Neigung von der Behälterwand zum zentralen Bodenbereich hin aufweist. Auf diese Weise wird auch bei Standardtankreaktoren mit ebenem Boden ein zuverlässiger Feststoffabzug gewährleistet.

## Beschreibung

Die Erfindung betrifft einen Bioreaktor zur Behandlung von organikhaltigen Schlammsuspensionen, wie Abfällen oder Güllen, mit einem einen Behälterboden aufweisenden Behälter und einem im zentralen Bodenbereich angebrachten Feststoffabzug.

Derartige Bioreaktoren werden z. B. zur aeroben oder anaeroben biologischen Behandlung von Klärschlamm, organische Bestandteile enthaltendem Restmüll und Güllen aus der Tierhaltung verwendet. Beim Einsatz solcher Bioreaktoren zur Müllbehandlung wird der Müll in Vorbehandlungsschritten in eine pumpfähige Schlammsuspension überführt. Anschließend wird die Schlammsuspension in vorzugsweise als volldurchmischte Tankreaktoren ausgebildeten Bioreaktoren üblicherweise anaerob behandelt, d.h. vergoren. Dieses als Nassvergärung in Gärreaktoren bekannte Verfahren ist gängige Praxis bei der biologischen Abfallbehandlung.

Ein Teil der in diesen Schlammsuspensionen immer enthaltenden partikulären Feststoffe kann vor dem Gärreaktor nicht effektiv entfernt werden, da in den viskosen Schlämmen eine Separation, z. B. durch Sedimentation, nur schwer realisierbar ist. Erst nach dem anaeroben Abbau eines Großteils der organischen Verbindungen sinkt die Viskosität im Gärgut soweit, dass partikuläre Feststoffe in den Gärreaktoren sedimentieren und bei langzeitlicher Akkumulation den Reaktor weitgehend zusetzen können.

Die einfach erscheinende Lösung, Reaktoren mit einem kegelförmig oder sonst wie geneigten Boden zu bauen, so dass Sedimente zu einem zentralen tiefsten Punkt als definierter Entnahmestelle rutschen, hat sich in der Praxis der Abfallvergärung aus Rentabilitätsgründen nicht durchgesetzt, da die Kegelböden bei den in Betracht kommenden Reaktorgrößen in Folge ihrer notwendigen tragenden Funktion für den gesamten Reaktor sehr kostenintensiv sind.

In gleicher Weise sind betonierte, in den Boden eingelassene Trichterböden nicht erwünscht, da sie wegen der Wasser gefährdenden Reaktorinhalte ein aufwendiges Lekagesicherungssystem erfordern.

Gängige Praxis ist deshalb die Kompromisslösung eines bis zu einem Neigungswinkel von ca. 10° in das Betonfundament des Gärreaktors eingegossenen flachen Kegels. Wie z. B. in der nicht vorveröffentlichten deutschen Patentanmeldung 103 58 400.5 beschrieben, wird diese flache Neigung in Verbindung mit einer der Flüssigkeit im Reaktorbodenbereich aufgeprägten Rotationströmung dazu genutzt, die Geschiebebewegungen der Sedimente zum zentrischen tiefsten Bodenpunkt für die Entnahme durch Absaugung zu unterstützen. Für Reaktoren mit großem Durchmesser und entsprechendem Behälterumfang bedeutet das einen nicht unerheblichen Investitionsaufwand und Energieverbrauch, um die Rotationströmung unter den beschriebenen Bedingungen aufrechtzuerhalten. Außerdem ist die Führung der Reaktorentnahmeleitungen im Bodenbereich und durch die Bodenplatte nicht unproblematisch.

Der vorliegenden Erfindung liegt die Aufgabe zugrunde, einen Bioreaktor der eingangsgenannten Art derart auszugestalten, dass auf wirtschaftliche Weise ein Abzug der partikulären Feststoffe vom Bioreaktor ermöglicht wird.

Diese Aufgabe wird erfindungsgemäß dadurch gelöst, dass im Bodenbereich ein nichttragender konischer Einsatz angeordnet ist, welcher eine Neigung von der Behälterwand zum zentralen Bodenbereich hin aufweist.

Wesentlich an der Erfindung ist also, dass die gewünschte Neigung zwischen Behälterwand und dem zentralen Bereich des Behälterbodens durch Einbringung eines nichttragenden, konischen Formteils im Bodenbereich eines Flachbodenreaktors realisiert wird. Dabei kann der Einsatz durch Anordnung passender Formsteine, durch Ausschäumen mit Auflage einer erosionsfesten Deckschicht oder als Gesparretragwerk mit konischer Auflage gestaltet werden. Die Auswahl hängt von der Reaktorgröße und den lokal kostengünstig verfügbaren und verarbeitbaren Materialien ab.

Gemäß einer besonders bevorzugten Ausgestaltung weist der Einsatz eine Oberfläche oder eine Auflage mit geringer Rauhigkeit und hoher Erosionsfestigkeit auf, um die Sedimentbewegung zum tiefliegenden Zentrum des Beckenbodens ohne Standzeitbeeinträchtigung langzeitlich auszuhalten.

Eine kraftschlüssige und tragende Verbindung des Einsatzes zur Behälterwand ist nicht erforderlich. Sofern keine Formgestaltung durch Formmaterialien erfolgt, ist davon auszugehen, dass die Freiräume im Gesparre im Laufe der Zeit mit Sediment und ausgefaultem Material zugesetzt werden. Konstruktiv sollte die Ausführung der Gestalt erfolgen, dass keine Taschen gebildet werden, in denen sich Biogas ansammeln und zu Auftreiben der Konstruktion führen kann. Gebildetes Biogas sollte unter der konischen Auflage aufperlen und längs des Spaltes zwischen Auflage und Behälterwand in den Behälterraum oberhalb des Einsatzes austreten können.

Bevorzugt weist der Einsatz eine Neigung von ca. 20° bis ca. 40° auf. Derartige Bodenneigungen lassen sich in der Praxis bei Bioreaktoren mit einem Reaktionsvolumen von 1000 bis 10000 m³ und Behälterdurchmessern zwischen 10m und 25m realisieren.

Es ist jedoch auch eine Ausschrägung lediglich des Eckbreiches zwischen Behälterwand und Behälterboden möglich, die dabei zweckmäßig unter 45° Neigung erfolgt, so dass ein wandnaher Bodenring überdeckt wird, dessen Breite der Konushöhe an der Behälterwand entspricht.

Mit diesen Maßnahmen ist eine wesentliche Intensivierung der Bewegung von sedimentiertem Material entlang der schiefen Ebene gewährleistet. Durch eine interne Schlaufenströmung über ein zentrales Leitrohr zur Mischung des Reaktorinhalts und Überlagerung einer Rotationströmung im Bodenbereich kann die Bewegung des sedimentierten Materials entlang der schiefen Ebene noch unterstützt werden. Ein Reaktor mit Schlaufenströmung ist beispielsweise in der DE 197 25 823 A1 beschrieben. In der nicht vorveröffentlichten deutschen Patentanmeldung 103 58 400.5 ist die Überlagerung einer Rotationsströmung im Bodenbereich des Reaktors dargestellt.

Dabei lassen sich für die genannten hydraulischen Reaktormischsysteme die energetischen Betriebskosten minimieren, da die Flüssigkeitsbewegung als alleiniges Vehikel zur Sedimentbewegung wie in Behältern mit Flachboden oder minimaler Neigung bis 10° für die erfindungsgemäße Ausführung in Folge der durch Schwerkraft bedingten Zusatzbewegung der Partikel reduziert werden kann.

Vorzugsweise sind zur hydraulischen Induzierung der bodennahen Flüssigkeitszirkulation Saugleitungen in der Behälterwand oberhalb der wandseitigen Konushöhe vorgesehen. Außerdem sind zweckmäßigerweise in einem Anstand von 0,5 bis 1,5 m oberhalb der wandseitigen Konushöhe Tangentialdüsen zur Einbringung einer Flüssigkeitsströmung in den Behälter angebracht.

Der konische Einsatz wird gemäß einer bevorzugten Ausgestaltung derart ausgeführt, dass er in einem Radiusbereich von 0,5 bis 4 m vom Bodenmittelpunkt des Behälters endet. Dabei liegt die innere Konuskante zweckmäßigerweise soweit über dem Behälterboden, dass Absaugungsleitungen sowie sonstige prozessbedingt erforderliche Spül- und Versorgungsleitungen unter dem Konusrand hindurchgeführt werden können, um funktionsgerecht in den konusfreien Zentrumsbereich zu münden. Dazu werden diese Leitungen in Nähe des Behälterbodens radial nach Außen zur Behälterwand und über entsprechende Stutzen oder Durchbrüche durch diese nach Außen geführt. Damit entfällt die problematische und aufwendige Führung durch den Behälterboden.

Andererseits wird auch die Strömungsstörung im bodennahen Flüssigkeitsbereich vermieden. Zudem lässt sich das Absaugverhalten durch günstige strömungstechnische Rohrmündungsgestaltung gegenüber der bisherigen Praxis verbessern, die Gefahr von Verstopfungen und Klassierungseffekten durch Vermeidung von Krümmern und aufwärts gerichteten Rohrstrecken im Behälterbodenbereich reduzieren sowie Service und Wartung erleichtern. Dies führt insgesamt zu verbesserten Systemstandzeiten und verringertem Anlagenrisiko.

Ein wesentlicher Vorteil der Erfindung besteht darin, dass ein Standard-Tankreaktor mit flachem Betonboden ohne Durchbrüche als Gärreaktor eingesetzt werden kann. Dabei wird mit der Erfindung der Sedimenttransport zur zentralen Entnahme verbessert, der Energieaufwand zur Aufrechterhaltung einer aufgabenrelevanten Rotationsströmung minimiert und die Leitungsführung im Bodenbereich systemfreundlich gestaltet. Für Reaktoren der in Frage kommenden Größen zwischen 1000 und 10000 m³ Reaktionsvolumen ist es kostengünstiger, unter Beibehaltung der Standardfertigung die Reaktorhöhe um 2 bis 4 m zu vergrößern, um im Bodenbereich den erfindungsgemäßen konischen Einsatz unterbringen zu können, als aufwendige Tanksonderkonstruktionen mit integriertem tragenden geneigten Bodenbereich zu errichten.

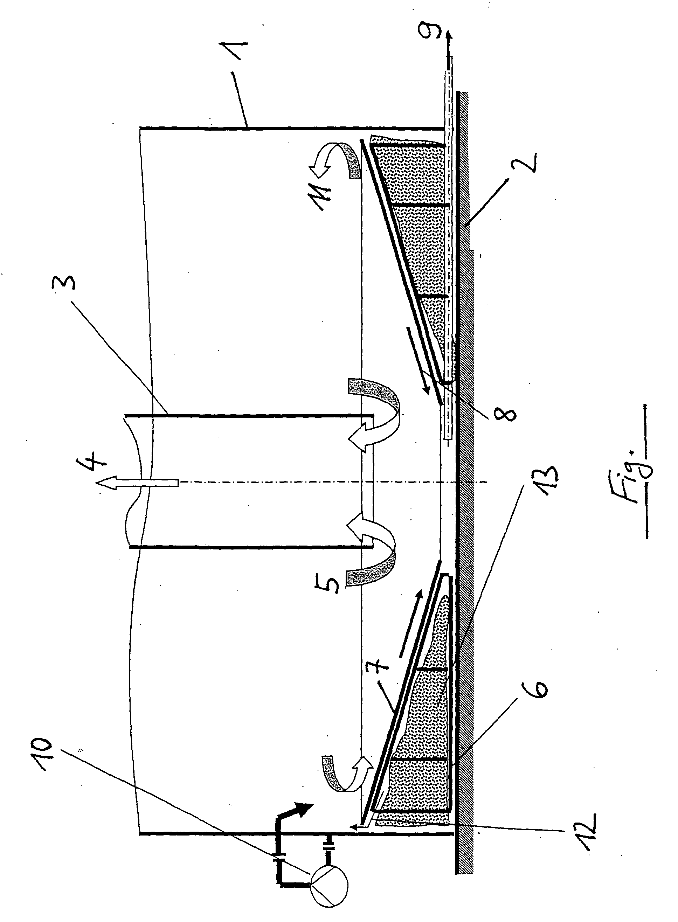

Im Folgenden soll die Erfindung anhand eines in der Figur schematisch dargestellten Ausführungsbeispiels näher erläutert werden:
In der Figur ist ein zylinderförmiger Gärreaktor mit einer Reaktorwand 1 und einem Reaktorboden 2 dargestellt. Der Gärreaktor weist ein zentrales Leitrohr 3 mit Biogasinjektion auf, das zur Umwälzung des Reaktorinhalts dient. Am unteren Ende des Leitrohres 3 wird Gärmedium angesaugt, gelangt als vertikaler Aufstrom 4 zum oberen Ende des Leitrohres 3 und kehrt als Schlaufenströmung 5 zum unteren Ende des Leitrohres 3 wieder zurück. Im Bodenbereich des Gärreaktors ist ein konischer nicht tragender Einsatz in Form eines Gesparretragrahmens mit einem Neigungswinkel von 20° bis 40° vorgesehen. Der Gesparretragrahmen 6 weist eine Auflage 7 auf, deren Oberfläche eine geringe Rauhigkeit und hohe Erosionsfestigkeit besitzt. Im Gärreaktor sich absetzende Feststoffe bewegen sich auf der Auflage 7 in Pfeilrichtung 8 in den zentralen Bodenbereich des Gärreaktors. Von dort werden sie über eine Entnahmeleitung 9 aus dem Gärreaktor abgezogen. Zur Überlagerung einer Rotationsströmung ist ein Injektionssystem 10 für eine bodennahe Flüssigkeitszirkulation vorgesehen. Dabei wird Flüssigkeit aus dem Gärreaktor abgesaugt und über Tangentialdüsen wieder in den Gärreaktorinnenraum eingetragen. Auf diese Weise wird eine horizontale Zirkulationsströmung 11 induziert. Unterhalb der Auflage 7 langzeitlich abgelagerte Sedimente und Gärrückstände 13 führen dazu, dass sich innerhalb des Gesparretragrahmens 6 Biogas bildet, welches abgeleitet werden muss. Hierzu ist vorgesehen, dass die Auflage 7 nicht bis zur Reaktorwand 1 reicht, sondern einen Spalt 11 für die Ausströmung von Biogas freilässt.

## Patentansprüche

1. Bioreaktor zur Behandlung von organikhaltigen Schlammsuspensionen, wie Abfällen oder Güllen, mit einem einen Behälterboden aufweisenden Behälter und einem im zentralen Bodenbereich angebrachten Feststoffabzug, **dadurch gekennzeichnet, dass** im Bodenbereich ein nichttragender, konischer Einsatz angeordnet ist, welcher eine Neigung von der Behälterwand zum zentralen Bodenbereich hin aufweist.

2. Bioreaktor nach Anspruch 1, **dadurch gekennzeichnet, dass** der Einsatz durch Anordnung von passenden Formsteinen gebildet ist.

3. Bioreaktor nach Anspruch 1, **dadurch gekennzeichnet, dass** der Einsatz durch Aufschäumen mit Auflage einer erosionsfesten Deckschicht gebildet ist.

4. Bioreaktor nach Anspruch 1, **dadurch gekennzeichnet, dass** der Einsatz als Gesparretragwerk mit konischer Auflage ausgebildet ist.

5. Bioreaktor nach Anspruch 3 oder 4, **dadurch gekennzeichnet, dass** zwischen Einsatz und Behälterwand ein Spalt zur Abgabe von unter der Auflage gebildetem Biogas in den Behälterraum vorgesehen ist.

6. Bioreaktor nach einem der Ansprüche 1 bis 5**,dadurch gekennzeichnet, dass** der Einsatz eine Neigung von ca. 20° bis ca. 40° aufweist.

7. Bioreaktor nach einem der Ansprüche 1 bis 5, **dadurch gekennzeichnet, dass** der Einsatz eine Ausschrägung des zwischen Behälterwand und Behälterboden gebildeten Eckbereichs mit einer Neigung unter 45° bildet.

8. Bioreaktor nach einem der Ansprüche 1 bis 7, **dadurch gekennzeichnet, dass** im Behälter Mittel zur Überlagerung einer Rotationsströmung der Schlammsuspension im Bodenbereich vorgesehen sind.

9. Bioreaktor nach Anspruch 8, **dadurch gekennzeichnet, dass** die Mittel zur Überlagerung der Rotationsströmung als in Behälterwandnähe angeordnete Düsen ausgebildet sind.

10. Bioreaktor nach Anspruch 9, **dadurch gekennzeichnet, dass** die Düsen 0.5 bis 1.5 m oberhalb der wandseitigen Konushöhe des Einsatzes angeordnet sind.

11. Bioreaktor nach einem der Ansprüche 1 bis 10, **dadurch gekennzeichnet, dass** der konische Einsatz derart ausgeführt ist, dass er in einem Radiusbereich von 0,5 bis 4 m vom Bodenmittelpunkt des Behälters endet.
